# EUROPEAN PATENT SPECIFICATION

(11) **EP 4 210 583 B1**
(45) Date of publication and mention of the grant of the patent: **06.11.2024**
(21) Application number: 21772802.1
(22) Date of filing: 07.09.2021
(51) Int. Cl.: A61B 8/00, A61B 8/08, G01N 29/28

(54) **METHOD OF PERFORMING ULTRASOUND MEASUREMENTS ON A HUMAN OR ANIMAL BODY**
VERFAHREN ZUR DURCHFÜHRUNG VON ULTRASCHALLMESSUNGEN AN EINEM MENSCHLICHEN ODER TIERISCHEN KÖRPER
PROCÉDÉ PERMETTANT D'EFFECTUER DES MESURES ULTRASONORES SUR UN CORPS HUMAIN OU ANIMAL

(30) Priority: 07.09.2020 EP 20194832
(43) Date of publication of application: 19.07.2023
(73) Proprietor: Rigshospitalet, 2100 Copenhagen Ø (DK)
(72) Inventor: SVENDSEN, Morten, Bo, Søndergaaard, 2800 Kgs. Lyngby (DK); GHULAM, Qasam, Mohammed, 2800 Kgs. Lyngby (DK); EIBERG, Jonas, 2860 Søborg (DK)
(74) Representative: Plougmann Vingtoft a/s
(86) International application number: PCT/EP2021/074559
(87) International publication number: WO 2022/049302

(56) References cited:
- US-A1- 2010 016 724
- US-A1- 2010 113 937
- US-A1- 2011 040 187
- US-A1- 2012 016 238

## Description

### FIELD OF THE INVENTION

The present invention relates to a method of performing ultrasound measurements on a human or animal body, and in particular it relates to such a method which can be used to obtain the pressure applied via an ultrasound probe used to perform the measurements.

### BACKGROUND OF THE INVENTION

Within the field of medical technology, ultrasound measurements are used for many different types of investigations on a human or animal body. Examples are for the investigation of abdominal aortic aneurisms (AAA) or for monitoring of the well-being and expected time of birth of a child.

Such measurements are typically performed by manually pressing an ultrasound transducer towards the skin of the body and moving the transducer over a region of interest while a resulting ultrasound image is studied on a display and possibly also recorded for further assessment. However, the pressure applied to the ultrasound transducer causes a compression of e.g. the AAA between the transducer and the spine, potentially resulting in an underestimation of AAA anterior-posterior diameter. Furthermore, since the transducer is held manually, the applied pressure will depend on the person performing the investigation, and it may also vary between two different investigations performed at different points in time. This can lead to erroneous conclusions being taken and for some types of investigations, this may cause erroneous or delayed treatment being initiated. It may also result in a conclusion that no treatment is needed even if it was.

Hence, an improved method of performing ultrasound measurements on a human or animal body would be advantageous.

US2012016238 may be considered to disclose a method according to the preamble of claim 1.

### OBJECT OF THE INVENTION

It is an object of the present invention to provide a method of performing ultrasound measurements on a human or animal body which gives more reliable results than known methods.

It is another object of the present invention to provide a method of obtaining a measure of the pressure of the ultrasound transducer on the object being scanned when performing ultrasound measurements on a human or animal body.

It is an object of at least some embodiments of the present invention to provide a method of performing ultrasound measurements on a human or animal body with which the final results are independent on the pressure applied to the ultrasound probe during the performance of the measurements.

It is another object of at least some embodiments of the present invention to provide a method of performing ultrasound measurements on a human or animal body with which the results are independent on the person performing the measurements.

It is a further object of the present invention to provide an alternative to the prior art.

In particular, it may be seen as an object of the present invention to provide a method of performing ultrasound measurements that solves the above mentioned problems of the prior art.

### SUMMARY OF THE INVENTION

Thus, the above-described object and several other objects are intended to be obtained in a first aspect of the invention by providing a method of performing ultrasound measurements on a human or animal body by use of an ultrasound probe, the method comprising:
- interpositioning of an ultrasound compatible and compressible elastic element between a tissue surface of the body and the ultrasound probe, the elastic element having predetermined elastic properties,
- performing the ultrasound measurements to obtain at least one ultrasound acquisition by sending ultrasound signals from the ultrasound probe into the body through the elastic element, the at least one ultrasound acquisition including:
   - a first region representing the compression of the elastic element being compressed by pressure applied with the ultrasound probe during the performance of the ultrasound measurements, and
   - a second region representing at least a part of the body under investigation,
- measuring the compression of the elastic element on the ultrasound acquisition, and
- based on the measured compression of the elastic element and a known correlation between elastic element compression and applied pressure, determining the pressure applied with the ultrasound transducer.

By "ultrasound compatible" is meant that ultrasound can be sent through the material without any significant scattering or dampening, and with a speed of sound resembling what it will be in human tissues.

By "compressible" is meant that a force applied by the ultrasound transducer will cause a deformation of the elastic element which is measureable on the ultrasound acquisition with a precision which is high enough to be used for the subsequent method steps.

By "an elastic element with predetermined elastic properties" is preferably meant that it is made from a material having a known relationship between compressive stress and strain. Hereby the measured elastic element deformation can be correlated to the applied pressure. The elastic element may e.g. have a linear relationship between stress and strain.

By such a method it is possible to adjust for the fact that different people may apply different amount of pressure on the ultrasound probe (interobserver variability), and that a person may not always apply the same pressure (intraobserver variability). Hereby it is possible to obtain measurement results which are standardized to provide results that are more reliable than what is possible with traditional ultrasound measurements on a human or animal body.

The elastic element is arranged freely on the tissue surface of the body. By "freely" is meant that it is unconstrained at all outer surfaces, with no adjacent frames or means of fixing the elastic element to e.g. electronic sensors. This provides the benefit that the elastic element can be used without extra sensors, but using the ultrasound probe without any add-ons, such as frames and mechanisms for measuring deformation, such as compression. The compression of the elastic element is determined directly on the ultrasound acquisition which is a simple and reliable way of determining the applied pressure. There is a further benefit that the deformation determined is the local deformation beneath the footprint of the ultrasound probe and thus not attenuated to the outside of the immediate footprint of the probe.

In some embodiments of the invention, the method further comprises a step of automatically correcting the part of the ultrasound acquisition being the second region to obtain standardized ultrasound measurement results which are independent on the applied pressure. Such automatic correction may take place in a computing unit connected to or forming part of the system used. The automatic correction will typically be based on numerical calculations possibly involving parameter characteristics for the type of investigations and/or measures related to the body under examination, such as weight or body mass index.

The obtained measure of the pressure may be presented visually or audibly during the performance of the ultrasound measurements. It could e.g. be presented on a display via a row of LEDs, a number, or a scale. Hereby it will be possible for the person performing the measurements to get an indication of the actual applied pressure and based thereon continuously adjust this to aim at a desired value. Such a desired value could e.g. be a record of a pressure used in a former measurement performed on the same body so that the results can be directly compared with larger precision than with known methods.

A similar method can also be used during training of persons performing ultrasound measurements. Such training could e.g. be used to give persons under education an interactive way of practising the performance of ultrasound measurements and which pressures that are appropriate. Hereby good skills within the field can be obtained faster than would normally be required.

In presently preferred embodiments of the invention, the elastic element is made from a material in which the speed of sound is in the range of 1200 to 1700 m/s, such as in the range of 1400 to 1600 m/s. This is around the speed of sound in human tissue which is about 1540 m/s.

In presently preferred embodiments of the invention, the elastic element is made from a material in which the attenuation of sound is below 3 dB/[mHz x cm], such as below 2 dB/[mHz x cm]. This is around the attenuation of sound in human tissue.

In general, the speed of sound as well as the attenuation of sound depend on the frequency of the ultrasound, but these values apply to the medical scanners typical used for the investigation of human bodies. By choosing a material for the elastic element with similar properties for the propagation of the ultrasound waves, imaging through the elastic element is enabled.

The elastic element may be made from a solid material selected from:
- ballistic gel and synthetic ballistic gel;
- hydrogels based on e.g. gelatine, agar, Psyllium husk, or Chia seeds;
- cryogels based on poly vinyl alcohol;
- polyurethane;
- poly vinyl chloride;
- silicones;
- glycerine softened polymers, such as thermoplastic starch.

It is possible to use any type of elastic material that can be traversed by ultrasound. In addition to the elastic properties of the material, the choice of material for a given application may depend on other parameter, such as price, how easy it is to clean if re-used, how stable the elastic properties are during longer term storage, and requirements related to disposal after use. E.g., hydrogels based on gelatine, agar, Psyllium husk, or Chia seeds will be biodegradable. Cryogel based on poly vinyl alcohol has the advantage that such a material is biodegrable (i.e. easily disposable), has stable mechanical properties, and the mechanical properties can be changed without additives. Furthermore, elements made from cryogel is easy to manufacture. Silicones are very non-biodegrable. They are very stable, and thus, counter-intuitively, considered one of the most "green" oil based polymers. Furthermore, silicones are easily manufactured and maintains the shape regardless of storage conditions within typical storage conditions. Glycerine softened polymers, such as thermoplastic starch, are biodegradable (i.e. easily disposable), and the plasticiser (glycerine) is very safe to humans as there is a low risk of negative influences, such as skin irritation, even with prolonged contact.

In alternative embodiments of the invention, the elastic element may comprise an inner cavity filled with liquid. Hereby it may be easier to avoid viscoelastic creep of the elastic element due to the applied pressure, whereby more precise measurements can be obtained. It may also be easier to find a material with a desired attenuation of sound and speed of sound for a given type of investigation. If the liquid is water, less waste material is obtained, as it does not require any special treatment after use. Other examples of liquids are physiological saline (H2O with NaCl 0.9%), glycerine, and polyethylene glycol. If the elastic element is provided with a closable and openable opening into the inner cavity, it is possible to adjust the volume and type of the liquid to a given application.

A method as described above may be at least a part of one of the following types of investigations: measurement of anatomy in the body, measurement of a foetus in the body, measurement of pathology in the body, measurement of abnormal/deformity in the body, and measurement of motion in the body, such as liquid motion.

In a second aspect, the present invention relates to a system for performing ultrasound measurements on a human or animal body, the system comprising:
- an ultrasound probe,
- an ultrasound compatible and compressible elastic element,
- a computing unit having access to known correlations between elastic element compression and applied pressure, and
wherein the system is adapted to perform a method as claimed in the claims.

By "computing unit" is meant a unit which can be used to determine the applied pressure based on measures of the compression of the elastic element and information about the predetermined elastic properties of the elastic element.

The elastic element is configured to be arranged freely on the tissue surface of the body during the performance of the ultrasound measurements.

The elastic element may be made from a material in which the speed of sound is in the range of 1200 to 1700 m/s, such as in the range of 1400 to 1600 m/s.

The elastic element may be made from a material in which the attenuation of sound is below 3 dB/[mHz x cm], such as below 2 dB/[mHz x cm].

The elastic element may be made from a solid material selected from:
- ballistic gel and synthetic ballistic gel;
- hydrogels based on e.g. gelatine, agar, Psyllium husk, or Chia seeds;
- cryogels based on poly vinyl alcohol;
- polyurethane;
- poly vinyl chloride;
- silicones;
- glycerine softened polymers, such as thermoplastic starch.

The elastic element may comprise an inner cavity filled with liquid.

The first and second aspects of the present invention may be combined. These and other aspects of the invention will be apparent from and elucidated with reference to the embodiments described hereinafter.

### BRIEF DESCRIPTION OF THE FIGURES

The method of performing ultrasound measurements on a human or animal body as well as a system for use in such a method according to the invention will now be described in more detail with regard to the accompanying figures. The figures show one way of implementing the present invention and is not to be construed as being limiting to other possible embodiments falling within the scope of the claims.
Figure 1 schematically shows how results from ultrasound measurements performed on a human or animal body can be influenced by the pressure applied with the ultrasound transducer.
Figure 2 schematically shows the measurements in figure 1 adjusted in accordance with the present invention.
Figure 3 shows an example of an ultrasound image obtained in accordance with the present invention.
Figure 4 shows results of measurements of the elastic properties of an elastic element for use in a method according to the present invention.
Figure 5 schematically shows an example of an elastic element that comprises an inner cavity filled with liquid.

### DETAILED DESCRIPTION OF AN EMBODIMENT

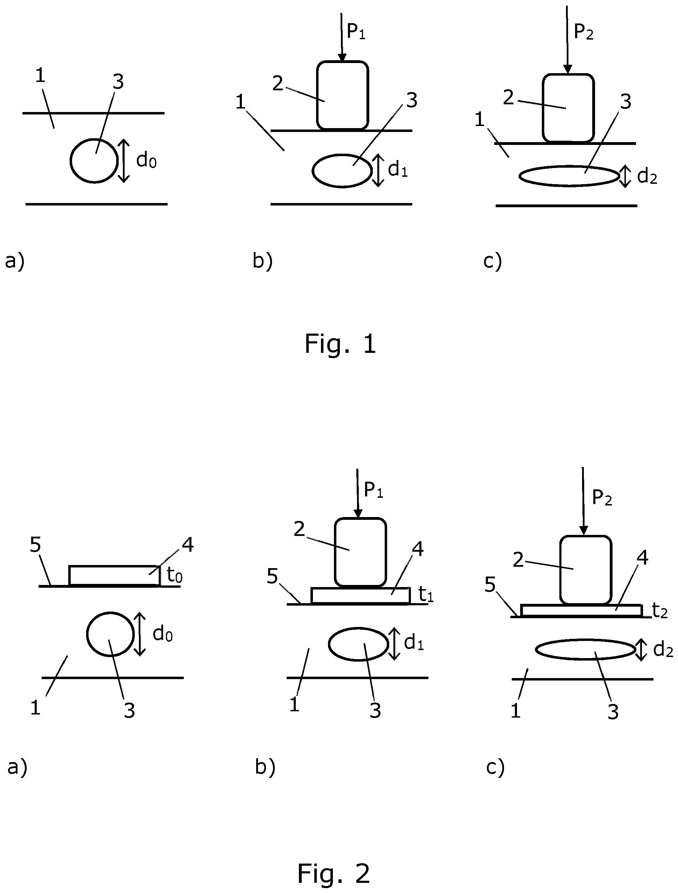

Figure 1 schematically shows how results from ultrasound measurements performed on a human or animal body 1 can be influenced by the pressure applied with the ultrasound transducer 2. Many different types of investigations can be performed by the invention. However, in order to better illustrate the overall principle, figure 1 shows a simple spherical cavity 3 and measurements of the size of that cavity 3. The deformations are exaggerated in the figures to better illustrate the invention. Figure 1.a shows a cross-sectional view of the spherical cavity 3 having a diameter d₀. Figure 1.b shows how measurements with an ultrasound transducer 2 with an applied pressure P₁ results in a measured diameter d₁ of the cavity 3 being deformed by the pressure P₁. Figure 1.c shows how measurements with an ultrasound transducer 2 with an applied pressure P₂ results in a measured diameter d₂ of the cavity 3 being deformed by the pressure P₂. The different pressures P₁ and P₂ could e.g. be applied by two different persons, or it could be applied by the same person at two different points in time. In both situations, this means that different conclusions may be made which can have serious consequences e.g. if the progress of a growing defect inside a patient is monitored.

Figure 2 schematically shows the measurements in figure 1 adjusted in accordance with the present invention by interpositioning of an ultrasound compatible and compressible elastic element 4 between a tissue surface 5 of the body 1 and the ultrasound probe 2. The elastic element 4 has predetermined elastic properties, and the ultrasound acquisition is obtained by sending ultrasound signals from the ultrasound probe 2 into the body 1 through the elastic element 4. The elastic element 4 is typically made from a material in which the speed of sound is in the range of 1200 to 1700 m/s, such as in the range of 1400 to 1600 m/s. The attenuation of sound is below 3 dB/[mHz x cm], such as below 2 dB/[mHz x cm]. These parameters are frequency dependent, and the numbers are here given in relation to the types of ultrasound transducers typically used for medical assessments.

For simplicity, the same deformations of the cavity 3 as in figure 1 are shown to take place even though in practise they may be slightly smaller since a part of the pressure is used for the compression of the elastic element 4. As seen from figures 2.b and 2.c, the differences in applied pressure also cause corresponding different compressions, t₁ and t₂, of the elastic element 4 during the performance of the ultrasound measurements.

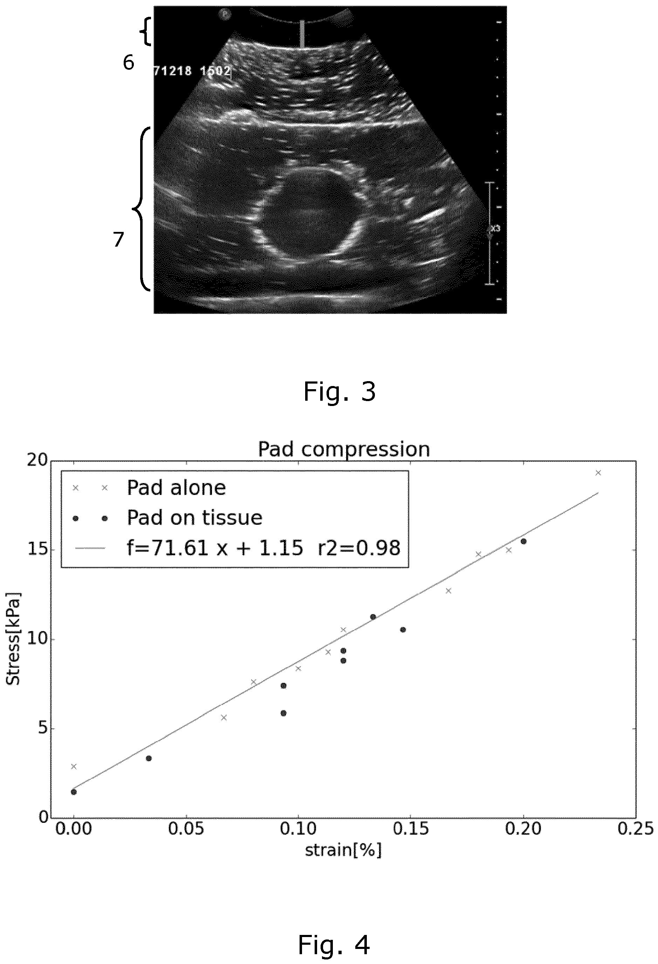

Figure 3 shows an example of an ultrasound image obtained in accordance with the present invention. The image is seen to include the first region 6 representing the thickness of the compressed elastic element 4 and the second region 7 representing a part of a human body 1 in the form of the region around and including a part of the aorta. The compression of the elastic element 4 on the ultrasound acquisition is measured, and based on the measured compression of the elastic element 4 and a known correlation between elastic element compression and applied pressure, it is possible to determine the pressure applied with the ultrasound transducer 2. As described above, for some embodiments of the invention, the measurements on the second region 7 can hereby be standardized to provide results that are independent on the applied pressure. Alternatively or in combination herewith, the obtained measure of the pressure may be presented visually or audibly during the performance of the ultrasound measurements. It could e.g. be presented on a display via a row of LEDs, a number, or a scale. Hereby it will be possible for the person performing the measurements to get an indication of the actual applied pressure and based thereon continuously adjust this to aim at a desired value as described in further details above.

The elastic properties of a given elastic element 4 to be used in the measurements as described above are predetermined so that this information can be used in the determination of the final results. As part of the studies leading to the present invention, it has been tested whether the type of surface on which the elastic element 4 is placed influences the elastic properties in a way that would be compromising to the quality of the results of the ultrasound measurements being performed. Figure 4 shows results of measurements of the elastic properties of an elastic element 4 for use in a method according to the present invention. In figure 4, the elastic element is referred to as "pad". The relationships between the stress and the strain were measured under conditions corresponding to real ultrasound measurements. In figure 4, the crosses represent measurements where the elastic element was placed on a table, i.e. a hard surface, and the dots represent measurements where the elastic element was placed on a pad of animal tissue. The line in the figure is a linear approximation of the results from the measurements made with the elastic elements placed on a hard surface. The slope of the line is the elastic modulus of the material, and as seen in the figure, this value will also be valid for the similar measurements with the softer surface in the form of tissue.

In summary, a method of performing ultrasound measurements on a human or animal body 1 in accordance with the present invention comprises the following steps:
- interpositioning of an ultrasound compatible and compressible elastic element 4 between a tissue surface 5 of the body 1 and the ultrasound probe 2, the elastic element 4 having predetermined elastic properties,
- performing the ultrasound measurements to obtain at least one ultrasound acquisition by sending ultrasound signals from the ultrasound probe 2 into the body 1 through the elastic element 4, the at least one ultrasound acquisition including:
- a first region 6 representing the compression of the elastic element 4 being compressed by pressure applied with the ultrasound probe 2 during the performance of the ultrasound measurements, and
- a second region 7 representing at least a part of the body 1 under investigation,
- measuring the compression of the elastic element 4 on the ultrasound acquisition, and
- based on the measured compression of the elastic element 4 and a known correlation between elastic element compression and applied pressure, determining the pressure applied with the ultrasound transducer 2, so that measurements on the second region 7 can be standardized to provide results that are independent on the applied pressure. The following is a list of examples of investigations that can be performed by the invention: measurement of anatomy in the body, measurement of a foetus in the body, measurement of pathology in the body, measurement of abnormal/deformity in the body, and measurement of motion in the body, such as liquid motion.

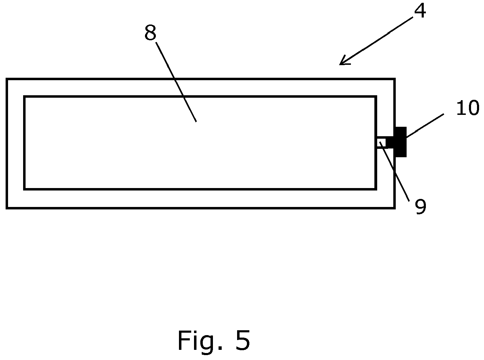

Figure 5 schematically shows an example of an elastic element 4 that comprises an inner cavity 8 filled with liquid. In this embodiment, a channel 9 extends from the inner cavity 8 towards an exterior surface of the elastic element 4. The channel 9 is provided with a plug 10 so that it is possible to remove the liquid, such as for replacing it with another liquid whereby it is possible to adjust the elastic properties of the elastic element 4 for a given application. The elastic properties of the elastic element 4 can also be varied by filling different amounts of liquid into the cavity as described above.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Furthermore, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. Method of performing ultrasound measurements on a human or animal body (1) by use of an ultrasound probe (2), the method comprising:
- interpositioning of an ultrasound compatible and compressible elastic element (4) between a tissue surface (5) of the body (1) and the ultrasound probe (2), the elastic element (4) having predetermined elastic properties,
- performing the ultrasound measurements to obtain at least one ultrasound acquisition by sending ultrasound signals from the ultrasound probe (2) into the body (1) through the elastic element (4), the at least one ultrasound acquisition including:
- a first region (6) representing the compression of the elastic element (4) being compressed by pressure applied with the ultrasound probe (2) during the performance of the ultrasound measurements, and
- a second region (7) representing at least a part of the body (1) under investigation,
- measuring the compression of the elastic element (4) on the ultrasound acquisition, and
- based on the measured compression of the elastic element (4) and a known correlation between elastic element compression and applied pressure, obtaining a measure of the pressure applied with the ultrasound transducer (2),
**characterised in that** the elastic element (4) is arranged freely on the tissue surface (5) of the body (1), meaning that the elastic element (4) is unconstrained at all outer surfaces thereof, with no adjacent frames or means of fixing the elastic element (4) to electronic sensors.

2. Method according to claim 1, further comprising a step of automatically correcting the part of the ultrasound acquisition being the second region (7) to obtain standardized ultrasound measurement results which are independent of the applied pressure.

3. Method according to any of the preceding claims, wherein the obtained measure of the pressure is presented visually or audibly during the performance of the ultrasound measurements.

4. Method according to any of the preceding claims, wherein the elastic element (4) is made from a material in which the speed of sound is in the range of 1200 to 1700 m/s, such as in the range of 1400 to 1600 m/s.

5. Method according to any of the preceding claims, wherein the elastic element (4) is made from a material in which the attenuation of sound is below 3 dB/[mHz x cm], such as below 2 dB/[mHz x cm].

6. Method according to any of the preceding claims, wherein the elastic element (4) is made from a solid material selected from:
- ballistic gel and synthetic ballistic gel;
- hydrogels based on e.g. gelatine, agar, Psyllium husk, or Chia seeds;
- cryogels based on poly vinyl alcohol;
- polyurethane;
- poly vinyl chloride;
- silicones;
- glycerine softened polymers, such as thermoplastic starch.

7. Method according to any of claims 1 to 5, wherein the elastic element (4) comprises an inner cavity (8) filled with liquid.

8. Method according to any of the preceding claims, wherein the method is at least a part of one of the following types of investigations: measurement of anatomy in the body, measurement of a foetus in the body, measurement of pathology in the body, measurement of abnormal/deformity in the body, and measurement of motion in the body, such as liquid motion.

9. System for performing ultrasound measurements on a human or animal body (1), the system comprising:
- an ultrasound probe (2),
- an ultrasound compatible and compressible elastic element (4) which is configured to be arranged freely on the tissue surface (5) of the body (1) during the performance of the ultrasound measurements so that the elastic element (4) is unconstrained at all outer surfaces thereof, with no adjacent frames or means of fixing the elastic element (4) to electronic sensors,
- a computing unit having access to known correlations between elastic element (4) compression and applied pressure, and
wherein the system is adapted to perform a method according to any of the preceding claims.

10. System according to claim 9, wherein the elastic element (4) is made from a material in which the speed of sound is in the range of 1200 to 1700 m/s, such as in the range of 1400 to 1600 m/s.

11. System according to any of claims 9 to 10, wherein the elastic element (4) is made from a material in which the attenuation of sound is below 3 dB/[mHz x cm], such as below 2 dB/[mHz x cm].

12. System according to any of claims 9 to 11, wherein the elastic element (4) is made from a solid material selected from:
- ballistic gel and synthetic ballistic gel;
- hydrogels based on e.g. gelatine, agar, Psyllium husk, or Chia seeds;
- cryogels based on poly vinyl alcohol;
- polyurethane;
- poly vinyl chloride;
- silicones;
- glycerine softened polymers, such as thermoplastic starch.

13. System according to any of claims 9 to 11, wherein the elastic element (4) comprises an inner cavity (8) filled with liquid.

## Patentansprüche

1. Verfahren zum Durchführen von Ultraschallmessungen an einem menschlichen oder tierischen Körper (1) unter Verwendung einer Ultraschallsonde (2), wobei das Verfahren Folgendes umfasst:
- Zwischenpositionieren eines ultraschallkompatiblen und komprimierbaren elastischen Elements (4) zwischen einer Gewebefläche (5) des Körpers (1) und der Ultraschallsonde (2), wobei das elastische Element (4) vorbestimmte elastische Eigenschaften aufweist,
- Durchführen der Ultraschallmessungen, um mindestens eine Ultraschallaufnahme zu erhalten, indem Ultraschallsignale von der Ultraschallsonde (2) durch das elastische Element (4) in den Körper (1) gesendet werden, wobei die mindestens eine Ultraschallaufnahme Folgendes beinhaltet:
- einen ersten Bereich (6), der die Kompression des elastischen Elements (4) darstellt, das durch Druck komprimiert wird, der mit der Ultraschallsonde (2) während der Durchführung der Ultraschallmessungen ausgeübt wird, und
- einen zweiten Bereich (7), der mindestens einen Teil des untersuchten Körpers (1) darstellt,
- Messen der Kompression des elastischen Elements (4) auf der Ultraschallaufnahme und
- Erhalten eines Maßes des mit dem Ultraschallwandler (2) ausgeübten Drucks auf Grundlage der gemessenen Kompression des elastischen Elements (4) und einer bekannten Korrelation zwischen der Kompression des elastischen Elements und dem angewendeten Druck,
**dadurch gekennzeichnet, dass** das elastische Element (4) frei auf der Gewebefläche (5) des Körpers (1) angeordnet ist, was bedeutet, dass das elastische Element (4) an allen Außenflächen davon nicht eingeschränkt ist, ohne benachbarte Rahmen oder Mittel zum Befestigen des elastischen Elements (4) an elektronischen Sensoren.

2. Verfahren nach Anspruch 1, ferner umfassend einen Schritt des automatischen Korrigierens des Teils der Ultraschallaufnahme, der der zweite Bereich (7) ist, um standardisierte Ultraschallmessergebnisse zu erhalten, die von dem angewendeten Druck unabhängig sind.

3. Verfahren nach einem der vorhergehenden Ansprüche, wobei das erhaltene Maß des Drucks während der Durchführung der Ultraschallmessungen visuell oder akustisch dargelegt wird.

4. Verfahren nach einem der vorhergehenden Ansprüche, wobei das elastische Element (4) aus einem Material gefertigt ist, in dem die Schallgeschwindigkeit im Bereich von 1200 bis 1700 m/s liegt, wie etwa im Bereich von 1400 bis 1600 m/s.

5. Verfahren nach einem der vorhergehenden Ansprüche, wobei das elastische Element (4) aus einem Material hergestellt ist, in dem die Schallabschwächung unter 3 dB/[mHz x cm] liegt, wie etwa unter 2 dB/[mHz x cm].

6. Verfahren nach einem der vorhergehenden Ansprüche, wobei das elastische Element (4) aus einem festen Material hergestellt ist, ausgewählt aus:
- ballistischem Gel und synthetischem ballistischem Gel;
- Hydrogele auf Grundlage von z. B. Gelatine, Agar, Flohsamenschalen oder Chiasamen;
- Kryogele auf Grundlage von Polyvinylalkohol;
- Polyurethan;
- Polyvinylchlorid;
- Silikone;
- mit Glyzerin weichgemachten Polymeren, wie etwa thermoplastischer Stärke.

7. Verfahren nach einem der Ansprüche 1 bis 5, wobei das elastische Element (4) einen inneren Hohlraum (8) umfasst, der mit Flüssigkeit gefüllt ist.

8. Verfahren nach einem der vorhergehenden Ansprüche, wobei das Verfahren mindestens Teil einer der folgenden Untersuchungsarten ist: Messung der Anatomie im Körper, Messung eines Fötus im Körper, Messung der Pathologie im Körper, Messung von Anomalien/Deformationen im Körper und Messung der Bewegung im Körper, wie etwa Flüssigkeitsbewegung.

9. System zum Durchführen von Ultraschallmessungen an einem menschlichen oder tierischen Körper (1), wobei das System Folgendes umfasst:
- eine Ultraschallsonde (2),
- ein mit Ultraschall kompatibles und komprimierbares elastisches Element (4), das dazu konfiguriert ist, während der Durchführung der Ultraschallmessungen frei auf der Gewebefläche (5) des Körpers (1) angeordnet zu werden, sodass das elastische Element (4) an allen Außenflächen davon nicht eingeschränkt ist, ohne benachbarte Rahmen oder Mittel zum Befestigen des elastischen Elements (4) an elektronischen Sensoren,
- eine Recheneinheit mit Zugriff auf bekannte Korrelationen zwischen der Kompression des elastischen Elements (4) und dem angewendeten Druck und wobei das System dazu ausgelegt ist, ein Verfahren nach einem der vorhergehenden Ansprüche durchzuführen.

10. System nach Anspruch 9, wobei das elastische Element (4) aus einem Material gefertigt ist, in dem die Schallgeschwindigkeit im Bereich von 1200 bis 1700 m/s liegt, wie etwa im Bereich von 1400 bis 1600 m/s.

11. System nach einem der Ansprüche 9 bis 10, wobei das elastische Element (4) aus einem Material hergestellt ist, in dem die Schallabschwächung unter 3 dB/[mHz x cm] liegt, wie etwa unter 2 dB/[mHz x cm].

12. System nach einem der Ansprüche 9 bis 11, wobei das elastische Element (4) aus einem festen Material hergestellt ist, ausgewählt aus:
- ballistischem Gel und synthetischem ballistischem Gel;
- Hydrogele auf Grundlage von z. B. Gelatine, Agar, Flohsamenschalen oder Chiasamen;
- Kryogele auf Grundlage von Polyvinylalkohol;
- Polyurethan;
- Polyvinylchlorid;
- Silikone;
- mit Glyzerin weichgemachten Polymeren, wie etwa thermoplastischer Stärke.

13. System nach einem der Ansprüche 9 bis 11, wobei das elastische Element (4) einen inneren Hohlraum (8) umfasst, der mit Flüssigkeit gefüllt ist.

## Revendications

1. Procédé de réalisation de mesures ultrasonores sur un corps humain ou animal (1) à l'aide d'une sonde à ultrasons (2), le procédé comprenant :
- l'intercalation d'un élément élastique (4) compatible avec les ultrasons et compressible entre une surface tissulaire (5) du corps (1) et la sonde à ultrasons (2), l'élément élastique (4) possédant des propriétés élastiques prédéfinies,
- la réalisation des mesures ultrasonores pour obtenir au moins une acquisition ultrasonore en envoyant des signaux ultrasonores de la sonde à ultrasons (2) dans le corps (1) à travers l'élément élastique (4), la au moins une acquisition ultrasonore comprenant :
- une première zone (6) représentant la compression de l'élément élastique (4) comprimé par la pression appliquée avec la sonde à ultrasons (2) durant la réalisation des mesures ultrasonores, et
- une seconde zone (7) représentant au moins une partie du corps (1) en cours d'examen,
- la mesure de la compression de l'élément élastique (4) sur l'acquisition ultrasonore, et
- sur la base de la compression mesurée de l'élément élastique (4) et d'une corrélation connue entre la compression de l'élément élastique et la pression appliquée, l'obtention d'une mesure de la pression appliquée avec le transducteur ultrasonore (2),
**caractérisé en ce que** l'élément élastique (4) est agencé librement sur la surface tissulaire (5) du corps (1), ce qui signifie que l'élément élastique (4) est libre toute contrainte sur toutes ses surfaces externes, sans cadres adjacents ni moyens de fixation de l'élément élastique (4) à des capteurs électroniques.

2. Procédé selon la revendication 1, comprenant en outre une étape de correction automatique de la partie de l'acquisition ultrasonore qui est la seconde zone (7) pour obtenir des résultats de mesure ultrasonore standardisés qui sont indépendants de la pression appliquée.

3. Procédé selon l'une quelconque des revendications précédentes, ladite mesure obtenue de la pression étant présentée visuellement ou de manière audible durant la réalisation des mesures ultrasonores.

4. Procédé selon l'une quelconque des revendications précédentes, ledit élément élastique (4) étant fabriqué à partir d'un matériau dans lequel la vitesse du son est comprise dans la plage de 1200 à 1700 m/s, par exemple dans la plage de 1400 à 1600 m/s.

5. Procédé selon l'une quelconque des revendications précédentes, ledit élément élastique (4) est fabriqué à partir d'un matériau dans lequel l'atténuation du son est inférieure à 3 dB/[mHz x cm], par exemple inférieure à 2 dB/[mHz x cm].

6. Procédé selon l'une quelconque des revendications précédentes, ledit élément élastique (4) étant fabriqué à partir d'un matériau solide choisi parmi :
- un gel balistique et un gel balistique synthétique ;
- des hydrogels à base de gélatine, d'agar, de cosses de psyllium ou de graines de chia, par exemple ;
- des cryogels à base d'alcool polyvinylique ;
- le polyuréthane ;
- le poly(chlorure de vinyle) ;
- des silicones ;
- des polymères ramollis à la glycérine, tels que l'amidon thermoplastique.

7. Procédé selon l'une quelconque des revendications 1 à 5, ledit élément élastique (4) comprenant une cavité interne (8) remplie de liquide.

8. Procédé selon l'une quelconque des revendications précédentes, ledit procédé faisant au moins partie de l'un des types d'examens suivants : mesure de l'anatomie dans le corps, mesure d'un foetus dans le corps, mesure d'une pathologie dans le corps, mesure d'une anomalie/d'une déformation dans le corps, et mesure d'un mouvement dans le corps, tel qu'un mouvement de liquide.

9. Système permettant de réaliser des mesures ultrasonores sur un corps humain ou animal (1), le système comprenant :
- une sonde à ultrasons (2),
- un élément élastique (4) compatible avec les ultrasons et compressible qui est configuré pour être agencé librement sur la surface tissulaire (5) du corps (1) durant la réalisation des mesures ultrasonores afin que l'élément élastique (4) soit libre de toute contrainte sur toutes ses surfaces externes, sans cadres adjacents ni moyens de fixation de l'élément élastique (4) à des capteurs électroniques,
- une unité de calcul possédant un accès aux corrélations connues entre la compression de l'élément élastique (4) et la pression appliquée, et
ledit système étant adapté pour réaliser le procédé selon l'une quelconque des revendications précédentes.

10. Système selon la revendication 9, ledit élément élastique (4) étant fabriqué à partir d'un matériau dans lequel la vitesse du son est comprise dans la plage de 1200 à 1700 m/s, par exemple dans la plage de 1400 à 1600 m/s.

11. Système selon l'une quelconque des revendications 9 à 10, ledit élément élastique (4) étant fabriqué à partir d'un matériau dans lequel l'atténuation du son est inférieure à 3 dB/[mHz x cm], par exemple inférieure à 2 dB/[mHz x cm].

12. Système selon l'une quelconque des revendications 9 à 11, dans lequel l'élément élastique (4) est fabriqué à partir d'un matériau solide choisi parmi :
- un gel balistique et un gel balistique synthétique ;
- des hydrogels à base de gélatine, d'agar, de cosses de psyllium ou de graines de chia, par exemple ;
- des cryogels à base d'alcool polyvinylique ;
- le polyuréthane ;
- le poly(chlorure de vinyle) ;
- des silicones ;
- des polymères ramollis à la glycérine, tels que l'amidon thermoplastique.

13. Système selon l'une quelconque des revendications 9 à 11, ledit élément élastique (4) comprenant une cavité interne (8) remplie de liquide.
